# EUROPEAN PATENT APPLICATION

(11) **EP 3 821 826 A1**
(43) Date of publication of application: **19.05.2021**
(21) Application number: 20207293.0
(22) Date of filing: 12.11.2020
(51) Int. Cl.: A61B 17/122, A61B 17/128

(54) **MULTI-PART LIGATION CLIP**

(30) Priority: 13.11.2019 US 201916682463
(71) Applicant: Covidien LP, Mansfield, MA 02048 (US)
(72) Inventor: BARIL, Jacob C., Norwalk, CT 06851 (US); LAPIERRE, Nicolette R., Windsor Locks, CT 06096 (US); PILLETERE, Roy J., North Haven, CT 06473 (US); DININO, Matthew A., Newington, CT 06111 (US); THOMAS, Justin, New Haven, CT 06512 (US); BROWN, Eric, Madison, CT 06443 (US)
(74) Representative: Maschio, Antonio

(57) **Abstract**

A multi-part ligation clip assembly includes a first beam, a second beam, and a spine that is positioned about the first and second beams to reinforce the clip assembly when the clip assembly is in a clamped position.

## Description

### FIELD

This disclosure is directed ligation clips for ligating tissue and, more particularly, to ligation clips for use in multi-fire clip appliers.

### BACKGROUND

Polymeric ligation clips typically include first and second beams that are coupled together at one end by a pivotable connection, e.g., living hinge, such that the first and second beams can be moved in relation to each other between open and clamped positions. The ligation clips can be applied to tissue endoscopically through a small diameter incision or through a small diameter cannula positioned through the incision to minimize trauma to a patient during a surgical procedure.

When polymeric clips are used to ligate large vessels or ducts, extreme stress is placed on the hinge of the clip which causes fatigue. If a clinician does not select a clip of proper size, the clip can become unclamped from the vessel or duct or fracture. This is dangerous for a patient.

### SUMMARY

In one aspect of the disclosure, a polymeric ligation clip assembly includes a first beam and a second beam, and a spine. The first beam defines a first longitudinal axis and has a first end portion, a second end portion, an inner first clamping surface extending between the first and second end portions, and an outer surface. The first end portion includes first coupling structure and the second end portion includes a first locking element. The second beam defines a second longitudinal axis and has a first end portion, a second end portion, an inner second clamping surface extending between the first and second end portions, and an outer surface. The first end portion of the second beam includes a second coupling structure and the second end portion of the second beam includes a second locking element. The second coupling structure is pivotably coupled to the first coupling structure to facilitate pivotable movement of the first beam in relation to the second beam from an open position to a clamped position. The spine is received about the first and second beams when the beams are in the clamped position to reinforce the first and second beams.

In another aspect of the disclosure, a ligation clip assembly includes a first beam, a second beam, and a spine. The first beam defines a first longitudinal axis and has a first end portion, a second end portion, and an outer surface. The outer surface defines a first longitudinal groove that extends along the first longitudinal axis. The second beam defines a second longitudinal axis and has a first end portion, a second end portion, and an outer surface. The outer surface of the second beam defines a second longitudinal groove that extends along the second longitudinal axis. The second beam is coupled to the first beam such that the first and second beams are movable from an open position to a clamped position in relation to each other. The spine is received about the first and second beams when the beams are in the clamped position to reinforce the first and second beams.

In yet another aspect of the disclosure, a ligation clip assembly includes a first beam, a second beam, and a U-shaped spine. The first beam defines a first longitudinal axis and has a first end portion, a second end portion, and an outer surface. The outer surface defines a first longitudinal groove that extends along the first longitudinal axis. The first end portion of the first beam includes a first coupling structure and the second end portion of the first beam includes a first locking element. The second beam defines a second longitudinal axis and has a first end portion, a second end portion, and an outer surface. The first end portion of the second beam includes a second coupling structure and the second end portion of the second beam includes a second locking element. The outer surface of the second beam defines a second longitudinal groove that extends along the second longitudinal axis. The second beam is coupled to the first beam such that the first and the second beams are movable from an open position to a clamped position in relation to each other. The U-shaped spine is received about the first and second beams when the beams are in the clamped position to reinforce the first and second beams. The U-shaped spine is received within the first and second longitudinal grooves and includes a first leg, a second leg, and back span interconnecting the first and second legs. The second coupling structure is adapted to be pivotably coupled to the first coupling structure to facilitate pivotable movement of the first beam in relation to the second beam from the open position to the clamped position.

In aspects of the disclosure, the spine is formed of a reinforcing material selected from the group consisting of spring steels, Nitinol, and titanium.

In some aspects of the disclosure, the first and second beams each define a longitudinal groove that extends along the outer surface of the respective first and second beam and receives the spine.

In certain aspects of the disclosure, the spine is U-shaped and includes a first leg, a second leg, and back span interconnecting the first and second legs.

In aspects of the disclosure, each of the longitudinal grooves defines an indentation and each of the first and second legs of the spine includes an inwardly extending projection that is received within one of the indentations to secure the spine onto the first and second beams.

In some aspects of the disclosure, each of the first and second legs of the spine have a distal end including an inwardly extending transverse portion and each of the longitudinal grooves defines a recess that receives the inwardly extending transverse portion to secure the spine to the first and second beams.

In aspects of the disclosure, the longitudinal grooves extend from a position adjacent the respective first and second locking elements to a proximal end of the respective first end portions of the first and second beams such that the spine is positioned about the first and second coupling structures.

In some aspects of the disclosure, the first locking element includes a hook member and the second locking member includes a receiver that defines a channel that extends about a distal end of the second beam.

In certain aspects of the disclosure, the hook member and the channel extend over an arc of greater than 180 degrees.

In aspects of the disclosure, the first and second beams are formed from a polymeric material.

Other features of the disclosure will be appreciated from the following description.

### BRIEF DESCRIPTION OF THE DRAWINGS

Various aspects of the disclosed multi-part polymeric ligation clip assembly are described herein below with reference to the drawings, wherein:
FIG. 1 is a side perspective view of the disclosed multi-part clip assembly with the clip in the clamped position;
FIG. 2 is a side perspective exploded view of the multi-part clip assembly shown in FIG. 1;
FIG. 3 is a side perspective view of first and second beams of the multi-part clip assembly shown in FIG. 2 with the first and second beams separated from each other;
FIG. 4 a side perspective view of the first and second beams of the multi-part clip assembly shown in FIG. 2 with the first and second beams coupled to each other in an open position positioned about a body vessel;
FIG. 5 a side perspective view of the multi-part clip assembly shown in FIG. 2 with the first and second beams coupled to each other and clamped about the body vessel and a spine of the clip assembly separated from the first and second beams;
FIG. 6 a side perspective view of the multi-part clip assembly shown in FIG. 2 with the first and second beams coupled to each other and clamped about the body vessel and a spine positioned on the first and second beams; and
FIG. 7 is a cross-sectional view taken along section line 7-7 of FIG. 6

### DETAILED DESCRIPTION

The disclosed multi-part polymeric ligation clip assembly will now be described in detail with reference to the drawings in which like reference numerals designate identical or corresponding elements in each of the several views. However, it is to be understood that the aspects of the disclosed ligation clip assembly are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure. In addition, directional terms such as front, rear, upper, lower, top, bottom, distal, proximal, and similar terms are used to assist in understanding the description and are not intended to limit the disclosure.

In this description, the term "proximal" is used generally to refer to that portion of the device that is closer to a clinician, while the term "distal" is used generally to refer to that portion of the device that is farther from the clinician. In addition, the term "endoscopic" is used generally to refer to endoscopic, laparoscopic, arthroscopic, and/or any other procedure conducted through a small diameter incision or cannula. Further, the term "clinician" is used generally to refer to medical personnel including doctors, nurses, and support personnel.

The disclosed multi-part ligation clip assembly includes a first beam, a second beam, and a spine, which are separate and distinct from each other. The spine is received on the first and second beams when the first and second beams are clamped about tissue after the first and the second beams are coupled together. The first beam has a first end portion including a first mating feature and the second beam has a first end portion including a second mating feature. The first and second mating features of the first and second beams can be selectively coupled together to facilitate pivotable movement of the beams of ligation clip assembly between open and clamped positions. The spine is placed about the first and second beams when the beams are moved to the clamped position to reinforce and secure the clip assembly in the clamped position.

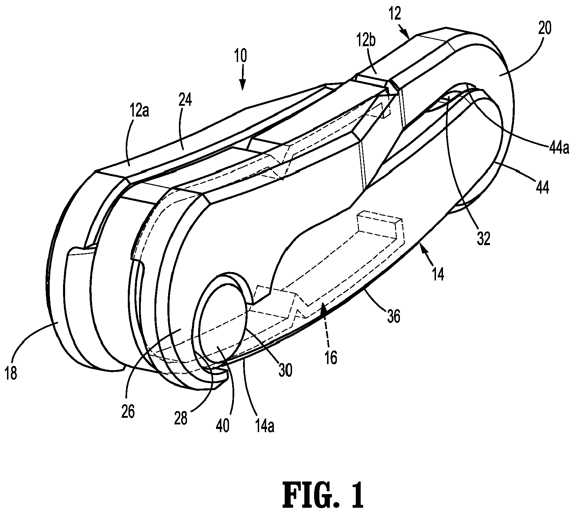

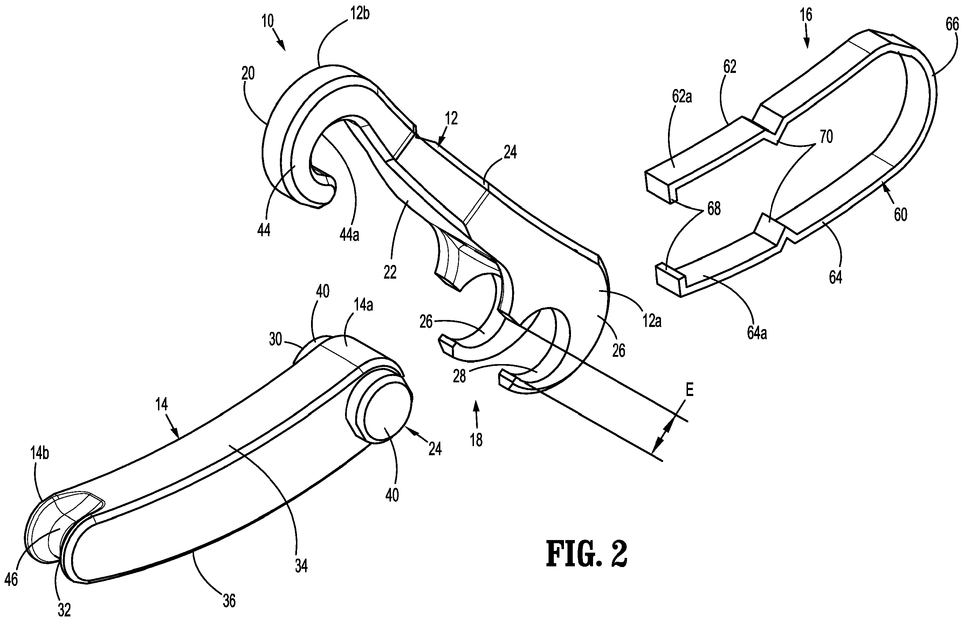

FIGS. 1-3 illustrate the disclosed multi-part clip assembly which is shown generally as clip assembly 10. The clip assembly 10 includes a first beam 12, a second beam 14, and a spine 16. The first beam 12 defines a longitudinal axis and has a first end portion 12a including first coupling structure 18, a second end portion 12b including a first locking element 20, an inner first clamping surface 22 (FIG. 2), and an outer surface 24. In certain aspects of the disclosure, the first coupling structure 18 includes a pair of curved, resilient arms 26 that define a cylindrical recess 28. The arms 26 are spaced from each other to receive the second beam 14.

The second beam 14 defines a longitudinal axis and has a first end portion 14a including second coupling structure 30, a second end portion 14b including a second locking element 32, an inner second clamping surface 34, and an outer surface 36. In certain aspects of the disclosure, the second coupling structure 30 includes a pair of spaced cylindrical hubs 40 that are received within the cylindrical recesses 28 defined by the arms 26 of the first coupling structure 18 to pivotably couple the first beam 12 to the second beam 14. When the cylindrical hubs 40 are received within the cylindrical recesses 28 of the arms 26 of the first coupling structure 18 (FIG. 1), the first beam 12 can pivot in relation to the second beam 14 between open and clamped positions. In the clamped position, (FIG. 1), the first clamping surface 22 of the first beam 12 is in juxtaposed alignment with the clamping surface 34 of the second beam 14. It is envisioned that other coupling structures can be used or provided to couple the first beam 12 with the second beam 14 to facilitate movement of the first beam 12 in relation to the second beam 14 between open and clamped positions, including, e.g., a pivot pin supported on one of the beams and openings or slots formed in the other of the beams.

Although not illustrated herein, the first and second clamping surfaces 22 and 34 may include tissue retention features such as protrusions and/or recesses to minimize slippage of the clip assembly 10 along tissue when the clip assembly 10 is clamped about tissue. Alternately, the first and second clamping surfaces 22, 34 can be flat or planar.

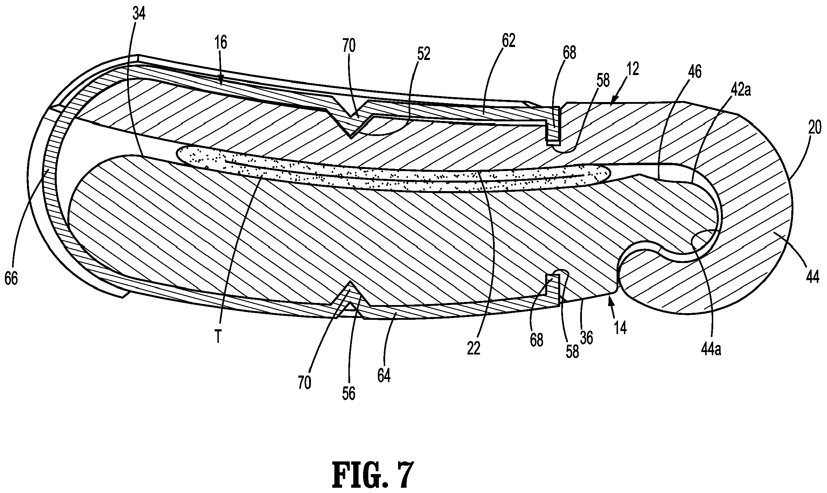

In aspects of the disclosed clip assembly 10, the first locking element 20 of the first beam 12 includes a latch member which is in the form of a hook member 44 and the second locking element 32 of the second beam 14 includes a latch member receiver in the form of a channel 46. The hook member 44 defines a circular recess 44a that receives a distal end of the second end portion 14b of the second beam 14. In some aspects of the disclosure, the hook member 44 extends along an arc of greater than 180 degrees (FIG. 7) and the channel 46 extends from the inner clamping surface 34 of the second beam 14, along the distal end of the second end portion 14b of the second beam 14, and onto the outer surface 36 of the second beam 14 over an arc of greater than 180 degrees. When the clip assembly 10 is pivoted to its clamped position as described in detail below, the hook member 44 passes through the channel 46, is deformed outwardly about the distal end of the second end portion 14b of the second beam 14, and flexes into the portion of the channel 46 positioned along the outer surface 36 of the second beam 14 to latch the first and second beams 12 and 14 in the clamped position. Alternately, the first and second locking elements 20 and 32 may assume a variety of different configurations to retain the ligation clip 10 in the clamped position.

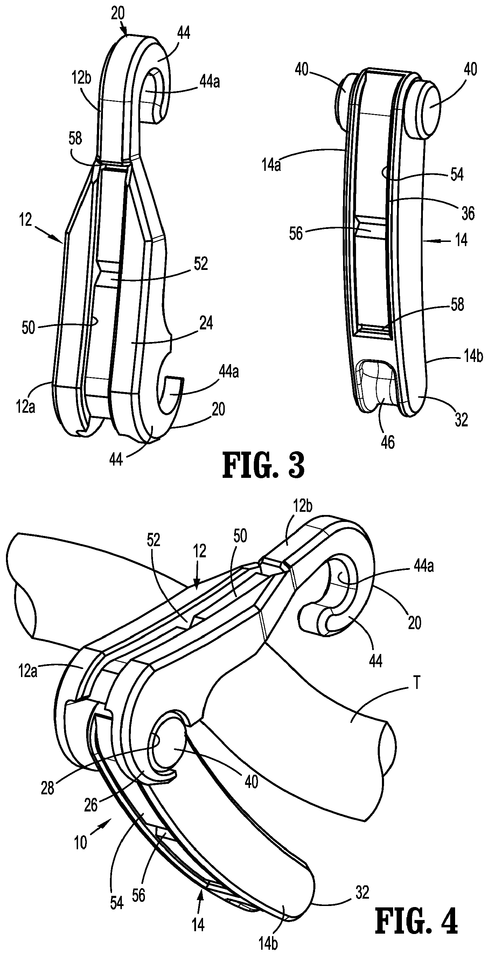

FIG. 3 illustrates the outer surfaces 24 and 36 of the first and second beams 12 and 14, respectively. The outer surface 24 of the first beam 12 defines a longitudinal channel or groove 50 that extends along the longitudinal axis of the first beam 12 from a position adjacent the first locking element 20 on the second end portion 12b of the first beam 12 to the proximal end of the first beam 12. The groove 50 includes at least one indentation 52. In some aspects of the disclosure, the indentation 52 is triangular in shape and is positioned centrally within the groove 50. Alternately, the indentation 52 may assume a variety of configurations and be positioned anywhere along the length of the groove 50.

Similarly, the outer surface 36 of the second beam 14 defines a longitudinal channel or groove 54 that extends along the longitudinal axis of the second beam 14 from a position adjacent the second locking element 32 on the second end portion 14b of the second beam 14 to the proximal end of the second beam 14. The groove 54 also includes at least one indentation 56 which also may be triangular in shape and positioned centrally within the groove 54. The ends of the first and second grooves 50 and 54 on the second end portion 12b and 14b of the first and second beams 12 and 14, respectively, each define a recess 58 that extends inwardly into the respective beam 12, 14 towards the respective clamping surface 22, 34.

FIG. 2 illustrates the spine 16 which is received within the first and second grooves 50 and 54 of the first and second beams 12 and 14, respectively, to reinforce the clip assembly 10 and to retain the clip assembly 10 in the clamped position. In some aspects, the spine 16 has a body 60 that has a substantially U-shape and includes a first leg 62, a second leg 64, and a curved back span 66. In other aspects, the spine 16 has a body 60 that is substantially C-shaped or V-shaped. Alternately, it is envisioned that the body 60 may assume a variety of configurations that are suitable for supporting the first and the second beams 12, 14 in the clamped position. Each of the first and second legs 62 and 64, respectively, has a distal end 62a, 64a, respectively, that includes an inwardly extending transverse portion 68 that extends in a direction towards the other leg of the spine 16. The back span 66 extends between and connects the proximal end of each of the legs 62 and 64 to each other. Each of the first and second legs 62 and 64 includes an inwardly extending projection 70 that is dimensioned and configured to be received within a respective one of the indentations 52 defined within the grooves 50 and 54 of the first and second beams 12 and 14. In certain aspects of the disclosure, the spine 16 is formed from a reinforcing material such as a spring steel including Nitinol or titanium. Alternately other materials having similar characteristics can be used to form the spine 16 including other metals, plastics, composites, or the like.

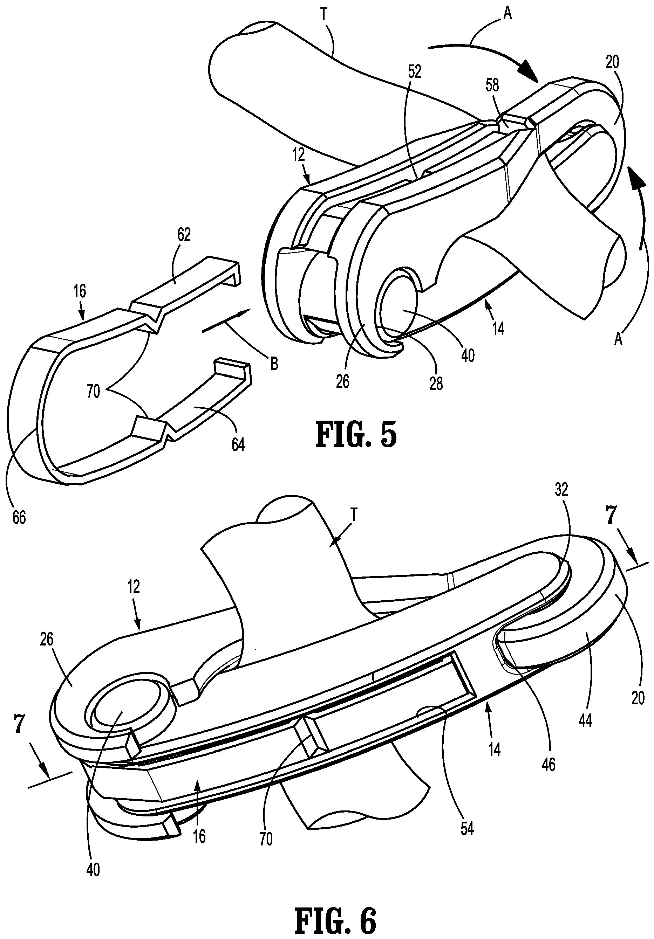

FIGS 4-7 illustrate the clip assembly 10 as the clip assembly 10 is positioned about tissue "T", e.g., a vessel or duct, and moved from the open position (FIG. 4) to the clamped position (FIG. 6). In FIG. 4, the first beam 12 is pivotably coupled to the second beam 14 by positioning the cylindrical hubs 40 formed on the first end portion 14a of the second beam 14 within the cylindrical recesses 28 of the arms 26 on the first end portion 12a of the first beam 12. In certain aspects of the disclosure, the arms 26 define an entryway "E" (FIG. 2) into the recesses 28 that has a width that is smaller than the diameter of the cylindrical hubs 40. As such, when the cylindrical hubs 40 are positioned within the cylindrical recesses 28 of the arms 26 through the entryway "E", the arms 26 are deformed outwardly to allow passage of the hubs 40. After the hubs 40 are received in the recesses 28, the arms 26 return to an undeformed state to partially enclose the hubs 40 and retain the hubs 40 within the recesses 28.

Once the first beam 12 is pivotably coupled to the second beam 14, the first and second beams 12 and 14 are positioned about the tissue "T" (FIG. 4) and the beams 12, 14 are pivoted in the directions indicated by arrows "A" to the clamped position. When the second ends 12a, 14a of the first and second beams 12 and 14 approach the clamped position (FIG. 5), the hooked member 44 on the second end 12b of the first beam 12 is received within the channel 46 on the second end 14b of the second beam 14. As the hooked member 44 moves through the channel 46, the hooked member 44 is deformed outwardly and passes over the distal end of the second beam 14. When the hooked member 44 passes over the distal face of the second beam 14, the hooked member 44 returns to its nondeformed state (FIG. 7) to latch the hooked member 44 within the channel 46 (FIG. 7) to secure the clamp assembly 10 in the clamped position.
After the first and second beams 12, 14 are clamped with the first and second locking elements 20 and 32 in a latched state, the spine 16 is slid onto the first end portions 12a and 14a of the first and second beams 12 and 14, respectively and into the grooves 50 and 54. The spine 16 is slid from the proximal end (first end portions 12a, 14a) of the first and second beams 12 and 14 about the first and second locking elements 20 and 32 and through the grooves 50 and 54 in the direction indicated by arrow "B" in FIG. 5. As the spine 16 is slid through the grooves 50 and 54 of the first and second beams 12 and 14, the legs 62 and 64 of the spine 16 will be deform outwardly until the projections 70 on the legs 62 and 64 of the spine 16 are received in the indentations 52 and 56 of the first and second beams 12 and 14 and the transverse portions 68 of the legs 62 and 64 of the spine 16 are received in the recesses 58 of the first and second beams 12 and 14 (FIG. 7). When this occurs, the legs 62 and 64 of the spine 16, due to the resilience of the material forming the spine 16, will snap onto the beams 12 and 14 and lock in place. Receipt of the projections 70 within the indentations 52 and the transverse portions 68 within the recesses 58 prevent the spine 16 from being inadvertently removed from about the beams 12 and 14. As discussed above, when the spine 16 is secured about first and second beams 12 and 14, the spine 16 helps to retain the beams 12 and 14 in the clamped position and reinforces the beams 12 and 14. When the spine 16 is received on the first and second beams 12 and 14, the spine 16 is positioned about the first and second coupling structures 18 and 30 to reinforce engagement of the coupling structures 18 and 30.

In embodiments, the first and second beams 12 and 14 of the ligation clip assembly 10 may be made at least in part of a resilient bioabsorbable polymeric material. Examples of suitable bioabsorbable polymeric materials include acetal polyoxymethylene (POM), polyethylene terephthalate (PET), polybutylene terephthalate (PBT), polyoxymethylene, polyetheretherketone (PEEK), polypropylene, and polyethylene or other thermoplastic materials having similar properties that can be injection-molded. The ligation clip 10 may also be made at least in part of a polymeric material or materials in combination with radiolucent metal alloys. Alternately, other materials may be used to form the ligation clip 10 including biocompatible metals, plastics and composites.

Persons skilled in the art will understand that the devices and methods specifically described herein and illustrated in the accompanying drawings are non-limiting exemplary aspects of the disclosed ligation clip. It is envisioned that the elements and features illustrated or described in connection with one exemplary embodiment may be combined with the elements and features of another without departing from the scope of the disclosure. As well, one skilled in the art will appreciate further features and advantages of the disclosure based on the above-described aspects of the disclosed ligation clip. Accordingly, the disclosure is not to be limited by what has been particularly shown and described, except as indicated by the appended claims.

The invention may be described by the following numbered paragraphs:
1. A ligation clip assembly comprising: a first beam defining a first longitudinal axis and having a first end portion, a second end portion, an inner first clamping surface extending between the first and second end portions, and an outer surface, the first end portion including first coupling structure and the second end portion including a first locking element; a second beam defining a second longitudinal axis and having a first end portion, a second end portion, an inner second clamping surface extending between the first and second end portions, and an outer surface, the first end portion of the second beam including a second coupling structure and the second end portion of the second beam including a second locking element, the second coupling structure adapted to be pivotably coupled to the first coupling structure to facilitate pivotable movement of the first beam in relation to the second beam from an open position to a clamped position; and a spine configured to be received about the first and second beams when the beams are in the clamped position to reinforce the first and second beams.
2. The ligation clip assembly of paragraph 1, wherein the spine is formed of a reinforcing material selected from the group consisting of spring steels, Nitinol, and titanium.
3. The ligation clip assembly of paragraph 1, wherein the first and second beams each define a longitudinal groove that extends along the outer surfaces of the respective first and second beam, the longitudinal groove receiving the spine.
4. The ligation clip assembly of paragraph 3, wherein the spine is U-shaped and includes a first leg, a second leg, and back span interconnecting the first and second legs.
5. The ligation clip assembly of paragraph 4, wherein each of the longitudinal grooves defines an indentation and each of the first and second legs of the spine includes an inwardly extending projection, the inwardly extending projections being received within the indentations to secure the spine onto the first and second beams.
6. The ligation clip assembly of paragraph 5, wherein each of the first and second legs of the spine have a distal end including an inwardly extending transverse portion and each of the longitudinal grooves defines a recess, the inwardly extending transverse portions being received within the recesses to secure the spine to the first and second beams.
7. The ligation clip assembly of paragraph 3, wherein the longitudinal grooves extend from a position adjacent the respective first and second locking elements to a proximal end of the respective first end portions of the first and second beams such that the spine is positioned about the first and second coupling structures.
8. The ligation clip assembly of paragraph 1, wherein the first locking element includes a hook member and the second locking member includes a receiver defining a channel, the channel extending about a distal end of the second beam.
9. The ligation clip assembly of paragraph 8, wherein the hook member and the channel extend over an arc of greater than 180 degrees.
10. The ligation clip of paragraph 1, wherein the first and second beams are formed from a polymeric material.
11. A ligation clip assembly comprising: a first beam defining a first longitudinal axis and having a first end portion, a second end portion, and an outer surface, the outer surface defining a first longitudinal groove that extends along the first longitudinal axis; a second beam defining a second longitudinal axis and having a first end portion, a second end portion, and an outer surface, the outer surface of the second beam defining a second longitudinal groove that extends along the second longitudinal axis, the second beam being coupled to the first beam such that the first and the second beams are movable from an open position to a clamped position in relation to each other; and a spine received about the first and second beams when the beams are in the clamped position to reinforce the first and second beams.
12. The ligation clip assembly of paragraph 11, wherein the spine is formed of a reinforcing material selected from the group consisting of spring steels, Nitinol, and titanium.
13. The ligation clip assembly of paragraph 12, wherein the spine is U-shaped and includes a first leg, a second leg, and back span interconnecting the first and second legs.
14. The ligation clip assembly of paragraph 13, wherein each of the first and second longitudinal grooves defines an indentation and each of the first and second legs of the spine includes an inwardly extending projection, the inwardly extending projections being received within the indentations to secure the spine onto the first and second beams.
15. The ligation clip assembly of paragraph 14, wherein each of the first and second legs of the spine have a distal end including an inwardly extending transverse portion and each of the first and second longitudinal grooves defines a recess, the inwardly extending transverse portions being received within the recesses to secure the spine to the first and second beams.
16. The ligation clip assembly of paragraph 11, wherein the first end portion of the first beam includes a first coupling structure and the second end portion of the first beam includes a first locking element, and the first end portion of the second beam includes a second coupling structure and the second end portion of the second beam includes a second locking element, the second coupling structure adapted to be pivotably coupled to the first coupling structure to facilitate pivotable movement of the first beam in relation to the second beam from the open position to the clamped position.
17. The ligation clip assembly of paragraph 16, wherein the first and second longitudinal grooves extend from a position adjacent the respective first and second locking elements to a proximal end of the respective first end portions of the first and second beams such that the spine is positioned about the first and second coupling structures
18. The ligation clip assembly of paragraph 16, wherein the first locking element includes a hook member and the second locking member includes a receiver defining a channel, the channel extending about a distal end of the second beam.
19. The ligation clip assembly of paragraph 18, wherein the hook member and the channel extend over an arc of greater than 180 degrees.
20. A ligation clip assembly comprising: a first beam defining a first longitudinal axis and having a first end portion, a second end portion, and an outer surface, the outer surface defining a first longitudinal groove that extends along the first longitudinal axis, the first end portion of the first beam including a first coupling structure and the second end portion of the first beam including a first locking element; a second beam defining a second longitudinal axis and having a first end portion, a second end portion, and an outer surface, the outer surface of the second beam defining a second longitudinal groove that extends along the second longitudinal axis, the first end portion of the second beam including a second coupling structure and the second end portion of the second beam including a second locking element, the second beam being coupled to the first beam such that the first and the second beams are movable in relation to each other from an open position to a clamped position, the second coupling structure adapted to be pivotably coupled to the first coupling structure to facilitate pivotable movement of the first beam in relation to the second beam from the open position to the clamped position; and a U-shaped spine configured to be received about the first and second beams when the beams are in the clamped position to reinforce the first and second beams, the U-shaped spine received within the first and second longitudinal grooves and including a first leg, a second leg, and back span interconnecting the first and second legs.

## Claims

1. A ligation clip assembly comprising:
a first beam defining a first longitudinal axis and having a first end portion, a second end portion, an inner first clamping surface extending between the first and second end portions, and an outer surface, the first end portion including first coupling structure and the second end portion including a first locking element;
a second beam defining a second longitudinal axis and having a first end portion, a second end portion, an inner second clamping surface extending between the first and second end portions, and an outer surface, the first end portion of the second beam including a second coupling structure and the second end portion of the second beam including a second locking element, the second coupling structure adapted to be pivotably coupled to the first coupling structure to facilitate pivotable movement of the first beam in relation to the second beam from an open position to a clamped position; and
a spine configured to be received about the first and second beams when the beams are in the clamped position to reinforce the first and second beams.

2. The ligation clip assembly of claim 1, wherein the spine is formed of a reinforcing material selected from the group consisting of spring steels, Nitinol, and titanium.

3. The ligation clip assembly of claim 1, wherein the first and second beams each define a longitudinal groove that extends along the outer surfaces of the respective first and second beam, the longitudinal groove receiving the spine; preferably wherein the spine is U-shaped and includes a first leg, a second leg, and back span interconnecting the first and second legs.

4. The ligation clip assembly of claim 3, wherein each of the longitudinal grooves defines an indentation and each of the first and second legs of the spine includes an inwardly extending projection, the inwardly extending projections being received within the indentations to secure the spine onto the first and second beams; preferably wherein each of the first and second legs of the spine have a distal end including an inwardly extending transverse portion and each of the longitudinal grooves defines a recess, the inwardly extending transverse portions being received within the recesses to secure the spine to the first and second beams.

5. The ligation clip assembly of claim 4, wherein the longitudinal grooves extend from a position adjacent the respective first and second locking elements to a proximal end of the respective first end portions of the first and second beams such that the spine is positioned about the first and second coupling structures.

6. The ligation clip assembly of any preceding claim, wherein the first locking element includes a hook member and the second locking member includes a receiver defining a channel, the channel extending about a distal end of the second beam; preferably wherein the hook member and the channel extend over an arc of greater than 180 degrees.

7. The ligation clip of any preceding claim, wherein the first and second beams are formed from a polymeric material.

8. A ligation clip assembly comprising:
a first beam defining a first longitudinal axis and having a first end portion, a second end portion, and an outer surface, the outer surface defining a first longitudinal groove that extends along the first longitudinal axis;
a second beam defining a second longitudinal axis and having a first end portion, a second end portion, and an outer surface, the outer surface of the second beam defining a second longitudinal groove that extends along the second longitudinal axis, the second beam being coupled to the first beam such that the first and the second beams are movable from an open position to a clamped position in relation to each other; and
a spine received about the first and second beams when the beams are in the clamped position to reinforce the first and second beams.

9. The ligation clip assembly of claim 8, wherein the spine is formed of a reinforcing material selected from the group consisting of spring steels, Nitinol, and titanium; preferably wherein the spine is U-shaped and includes a first leg, a second leg, and back span interconnecting the first and second legs.

10. The ligation clip assembly of claim 9, wherein each of the first and second longitudinal grooves defines an indentation and each of the first and second legs of the spine includes an inwardly extending projection, the inwardly extending projections being received within the indentations to secure the spine onto the first and second beams.

11. The ligation clip assembly of claim 10, wherein each of the first and second legs of the spine have a distal end including an inwardly extending transverse portion and each of the first and second longitudinal grooves defines a recess, the inwardly extending transverse portions being received within the recesses to secure the spine to the first and second beams.

12. The ligation clip assembly of any of claims 8 to 11, wherein the first end portion of the first beam includes a first coupling structure and the second end portion of the first beam includes a first locking element, and the first end portion of the second beam includes a second coupling structure and the second end portion of the second beam includes a second locking element, the second coupling structure adapted to be pivotably coupled to the first coupling structure to facilitate pivotable movement of the first beam in relation to the second beam from the open position to the clamped position; preferably wherein the first and second longitudinal grooves extend from a position adjacent the respective first and second locking elements to a proximal end of the respective first end portions of the first and second beams such that the spine is positioned about the first and second coupling structures

13. The ligation clip assembly of claim 12, wherein the first locking element includes a hook member and the second locking member includes a receiver defining a channel, the channel extending about a distal end of the second beam.

14. The ligation clip assembly of claim 13, wherein the hook member and the channel extend over an arc of greater than 180 degrees.

15. A ligation clip assembly comprising:
a first beam defining a first longitudinal axis and having a first end portion, a second end portion, and an outer surface, the outer surface defining a first longitudinal groove that extends along the first longitudinal axis, the first end portion of the first beam including a first coupling structure and the second end portion of the first beam including a first locking element;
a second beam defining a second longitudinal axis and having a first end portion, a second end portion, and an outer surface, the outer surface of the second beam defining a second longitudinal groove that extends along the second longitudinal axis, the first end portion of the second beam including a second coupling structure and the second end portion of the second beam including a second locking element, the second beam being coupled to the first beam such that the first and the second beams are movable in relation to each other from an open position to a clamped position, the second coupling structure adapted to be pivotably coupled to the first coupling structure to facilitate pivotable movement of the first beam in relation to the second beam from the open position to the clamped position; and
a U-shaped spine configured to be received about the first and second beams when the beams are in the clamped position to reinforce the first and second beams, the U-shaped spine received within the first and second longitudinal grooves and including a first leg, a second leg, and back span interconnecting the first and second legs.
